Europäisches Patentamt

European Patent Office

Office européen des brevets

(11) Publication number: **0 304 845**
**A2**

# EUROPEAN PATENT APPLICATION

(21) Application number: 88113624.6

(22) Date of filing: 22.08.88

(51) Int. Cl.⁴: **C12Q 1/68**

(30) Priority: 28.08.87 US 90789

(43) Date of publication of application:
01.03.89 Bulletin 89/09

(84) Designated Contracting States:
AT BE CH DE ES FR GB GR IT LI NL SE

(71) Applicant: **PROFILE DIAGNOSTIC SCIENCES INC.**
**400 Valley Road**
**Warrington Pennsylvania(US)**

(72) Inventor: **Wang, Chia-Gee**
**44 Glenwood Road**
**Millwood New York(US)**

(74) Representative: **Baillie, Iain Cameron et al**
**c/o Ladas & Parry Isartorplatz 5**
**D-8000 München 2(DE)**

(54) Method and kit for assaying gene expressions.

(57) A method of assaying gene expressions comprises linking mRNA molecules, including certain target mRNA molecules to be assayed, to a solid substrate; contacting said solid substrate with an aqueous suspension of labelled microbeads to which gene probe molecules are linked, said gene probe molecules comprising sequences which hybridize with sequences comprised by and characteristic of said certain target mRNA molecules to be assayed, thereby selectively linking said labelled microbeads to said certain target mRNA molecules to be assayed which are linked to said substrate; separating the solid substrate from microbeads which are not linked to said target mRNA molecules; and determining the presence or quantity of said labelled, linked microbeads; thereby selectively assaying said gene expressions.

EP 0 304 845 A2

## METHOD AND KIT FOR ASSAYING GENE EXPRESSIONS

The invention relates to a method of assaying or determining the presence or quantity of gene expressions, and to a kit useful for such assays.

There is a great interest in detecting the expressions of specific genes because of the value of such information in genetic research and medical research into the causes, diagnosis and treatment of diseases such as cancer.

The expressions of genes result from the complex procedures by which proteins are synthesized from the genetic information in DNA. Messenger RNA (mRNA) molecules are formed by transcription of the expressed DNA. The mRNA is translated to form proteins. Thus, an analysis which determines the presence or quantity of mRNA molecules which are the product of a particular gene provides the most direct assay of the gene expressions.

Important analytical methods for the detection of biological molecules include methods which utilize antibodies which selectively combine with the target molecule, i.e. the substance to be detected, and methods which use gene probes which hybridize with DNA or RNA target chains or molecules. The antibodies or gene probes are referred to as "detectors". When they combine with the target molecule, a means of reporting the conjugate is needed. Such means, called the "reporter" can be the addition of a label or tag to the antibody or gene probe, such as the well-known use of radioactive atoms or fluorescent molecules. The extensive prior art relating to the use of antibodies is discussed in U.S. Patents 4,454,233 granted June 12, 1984 and 4,436,826 granted March 13, 1984, both granted to the present inventor Chia-Gee Wang, the disclosures of both patents being incorporated herein by reference. These patents disclose the use of microspheres to couple with the antibody detector, and the use of tagging or labelling reporters which can be detected by a number of methods including X-ray fluorescence. U.S. Patent No. 4,663,277 granted on May 5, 1987 also to the present inventor, the disclosure of which is incorporated herein by reference, describes a method for the detection of viruses or proteins using a solid phase amplification technique. The target viruses or proteins in a specimen are contacted with a solid substrate coated with an antibody which forms a complex with the target, and microspheres coated with antibody are then bound to the target complex. The detection sensitivity is amplified by the labelled microspheres. The use of X-ray fluore-scence analytical techniques is disclosed, enabling simultaneous determination of different kinds of targets.

The use of gene probes is described generally in "Molecular Biology of the Gene", by James D. Watson et al, Fourth Edition, 1987, Vol. I, pages 608-611 and 633, the disclosure of which is incorporated herein by reference. It is disclosed that a gene probe can be obtained from RNA or DNA and can search out DNA or RNA segments of almost an exact complement to certain sequences in bacterial colonies. The techniques using such probes with radio-active labels in the Southern blotting and Northern blotting methods are described. Detailed techniques relating to cloning, hybridization and radioactive gene probes are described in the text "Molecular Cloning", T. Maniatis et al., Cold Spring Harbor Laboratory, 1982, the disclosure of which is incorporated herein by reference.

According to the present invention there is provided a method of assaying gene expressions, which comprises linking mRNA molecules, including certain target mRNA molecules to be assayed, to a solid substrate; contacting said solid substrate with an aqueous suspension of labelled microbeads to which gene probe molecules are linked, said gene probe molecules comprising sequences which hybridize with sequences comprised by and characteristic of said certain target mRNA molecules to be assayed, thereby selectively linking said labelled microbeads to said certain target mRNA molecules to be assayed which are linked to said substrate; separating the solid substrate from microbeads which are not linked to said target mRNA molecules; and determining the presence or quantity of said labelled, linked microbeads; thereby selectively assaying said gene expressions.

Also provided in accordance with the invention is a kit for assaying gene expressions in aqueous specimens containing mRNA molecules including certain target mRNA molecules to be assayed, which comprises as individual components (a) a solid substrate having conjugated thereto RNA or DNA polymers capable of hybridizing with 3′ poly (A) tails of said mRNA molecules; and (b) an aqueous suspension of labelled microbeads to which are linked a plurality of RNA or DNA polymers, each of said linked RNA or DNA polymers being covalently linked at one end to the surface of said labelled micro-bead and being linked at another end to an end of a RNA or DNA gene probe molecule, said gene probe molecule comprising an anti-sense sequence capable of hybridizing with a sense sequence comprised by and characteristic of said certain target mRNA molecules to be assayed.

By using microbeads as the reporter element in a technique of "Solid Phase Amplification" (SPA) which employs a gene probe, the new technique referred to herein as "SPAp" can readily reach sub-atto mole sensitivity in the analysis of target sequences. The SPAp system can simultaneously detect several different mRNA targets from a very small specimen in semi-liquid-phase hybridization and report the result quantitatively. An internal standard can be used to increase accuracy.

In an embodiment of the invention, tumor tissues can be analyzed with respect to their expressions of oncogenic sequences so that a disease-oriented diagnosis can be obtained rapidly and conveniently. The SPAp system can be developed to serve a variety of biomedical needs and interests.

FIG. 1 is a schematic representation of one embodiment of the assay of the present invention.

FIG. 2 is a schematic representation of another embodiment of the assay of the present invention.

Monoclonal antibodies (mAb) and gene probes are two extremely powerful detectors developed in recent years to assay molecules of biomedical interest. These detectors can be very specific in recognizing a target ligand and the probe reagents can be prepared for a variety of purposes. To serve in a diagnosis, these versatile and specific detectors also need a convenient reporter to indicate their conjugations. The most popular current reporters include radioactive atoms used in radioimmunoassay (RIA) or in dot blot hybridization; fluorescent molecules used in fluorescent immunoassay (FIA); and enzyme molecules used in enzyme linked immunosorbant assay (ELISA) or in biotinylation of gene probes. The SPA reporter used in the present invention with a gene probe detector, the SPAp system, includes the advantage of high sensitivity, and further, simultaneous detecting and reporting of multiple targets in a single procedure can be obtained in an embodiment of the present invention when the reporters are detected by X-ray fluorescence analysis.

In SPA for biomedical diagnosis, the reporting elements are chemically separated from the detecting element. A well known example that illustrates the SPA principle is the immunogold procedure. Examined under an electron microscope, the Ab coated gold micro-particles can be counted one at a time, therefore they can report essentially the presence of single target ligand. Extensive tissue preparation and other restrictions, however, render the immunogold method impracticable for most diagnostic services. The SPAp system of the present invention does not require use of an electron microscope, nor does it employ solid phase-hybridization. The preferred SPA reporters consist of a variety of trace elements of the periodic table, added to microspheres as a "mobile solid phase. These trace particles are covalently linked with various gene probes as detectors. In a diagnosis, the assay sensitivity depends on the reporting as well as the detecting functions. Since the SPA reporters can be sensitive to the level of single target ligand, a value far surpassing any detector sensitivity with finite conjugating affinity, the assay therefore can be designed to achieve an optimal detecting function with regard to accuracy, reliability, speed, convenience, and cost.

The assay method of the present invention can be applied to the analysis of many expressions of genes, where the target molecules contain characteristic sequences of nucleotides, that is, "sense" sequences which are characteristic of those molecules, for which corresponding complement gene probe molecules can be obtained or designed for recognition of and hybridization with such characteristic sequences.

Except for specialized cells which produce large amounts of a particular protein, most cells contain over 1,000 different RNA molecules. Therefore, a specimen will contain a great multitude of different gene expressions. In the method of the present invention, a specimen which may contain such large numbers of different mRNA molecules is brought into contact with a solid substrate to which different mRNA molecules are linked. Among these are certain target mRNA molecules desired to be assayed, and these are identified or detected by gene probe molecules comprising sequences which hybridize with sequences characteristic of those mRNA molecules which are to be assayed. An aqueous suspension of labelled microbeads to which such gene probe molecules are linked is applied to contact the solid substrate to which the mRNA molecules are linked. The gene probe molecules hybridize with the mRNA molecules which are to be assayed so that the labelled microbeads are selectively bound to those mRNA molecules desired to be assayed, which in turn are linked to the substrate. The solid substrate then is separated from microbeads which are not linked to said target mRNA molecules, such as by a washing procedure, the presence or the quantity of the labelled, linked microbeads is determined, and since such microbeads are essentially linked only to mRNA molecules which are to be assayed, this determination results in a selective assay of the gene expressions.

In a preferred embodiment of the present in vention, gene expressions are assayed by a method which comprises contacting an aqueous specimen containing mRNA molecules including certain target mRNA molecules to be assayed with a solid substrate having conjugated thereto RNA or DNA polymers which hybridize with 3' poly (A) tails of said mRNA molecules, thereby linking mRNA molecules to said solid substrate; separating said solid substrate from said aqueous specimen; contacting said solid substrate with an aqueous suspension of labelled microbeads to which are linked a plurality of RNA or DNA polymers, each of said linked RNA or DNA polymers being covalently linked at one end to the surface of said labelled microbead and being linked at another end to an end of a RNA or DNA gene probe molecule, said gene probe molecule comprising an anti-sense sequence capable of hybridizing with a sense sequence comprised by and characteristic of said certain target mRNA molecules to be assayed; hybridizing said gene probe molecules with said certain target mRNA molecules to be assayed, thereby selectively linking said labelled microbeads to said certain target mRNA molecules to be assayed which are linked to said solid substrate; separating the solid substrate from microbeads which are not linked to said target mRNA molecules; and determining the presence or quantity of said labelled, linked microbeads, thereby selectively assaying said gene expressions.

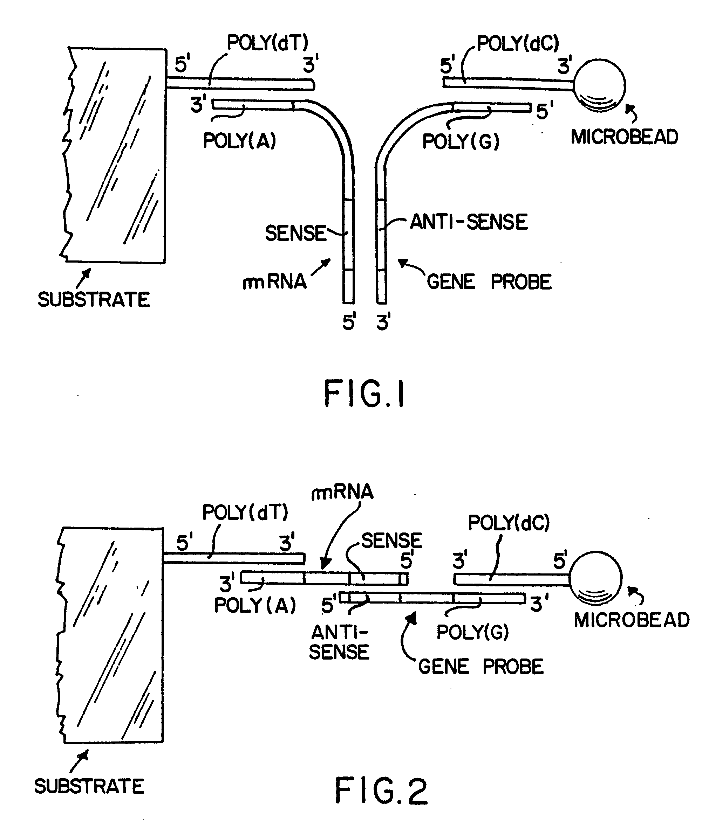

As is shown in FIG. 1, RNA or DNA polymers may be covalently linked at their 3′ ends to the labelled microbeads and will become linked at their 5′ ends to the 5′ ends of the gene probe molecules. In the configuration shown in FIG. 1, the nucleotides of the anti-sense sequence of the gene probe are complementary to the nucleotides of the sense sequence of the target mRNA. FIG. 2 illustrates an alternative configuration in accordance with the invention. In this embodiment the RNA or DNA polymers are covalently linked at their 5′ ends to the labelled microbeads and are linked at their 3′ ends to the 3′ ends of the gene probe molecules.

The mRNA molecules including the ones desired to be assayed may be linked to the solid substrate through linking with RNA or DNA polymers conjugated on the surface of the substrate. The conjugated polymers normally will be covalently linked to the substrate at their 5′ ends. Such polymers may be poly (dT) or poly (U) homopolymers.

When the gene probe molecules are linked to the microbeads by linking to the ends of RNA or DNA polymers, the polymers linked to the labelled microbeads are poly (X) homopolymers and the gene probe molecules are extended with poly (Y) homopolymers at the end which is linked to said polymers which are linked to said microbeads, wherein:

X is dC and Y is G, X is C and Y is G, X is dG and Y is C, X is G and Y is C, X is dC and Y is dG, X is C and Y is dG, X is dG and Y is dC or X is G and Y is dC.

The microbeads used in the present invention preferably are small latex beads such as those supplied by Polyscience, Inc. (Paul Valley Industrial Park, Warrington, PA 18976). A preferred size (diameter) of the microbeads is 0.3 micrometers or less. To improve sensitivity, is preferred that the microbeads should be 0.25 micrometers or smaller. The microbeads preferably are not smaller than 0.05 micrometers since below this size the signals obtained in detection steps become weak.

The microbeads are labelled such as by con taining radioactive atoms or elements which can be measured by optical, magnetic, acoustic or fluorescent methods. It is preferred that the labels should be metal elements or compounds which are stable in solution and detectable by X-ray fluorescence. Preferably, the labels are heavy elements having an atomic weight in excess of 50 so that their X-ray fluorescence properties are not strongly attenuated in the air. Examples of such elements are Fe, Ni, Cu, Co and the like.

The solid substrate may be composed of any material to which the mRNA molecules can be linked, and preferably is made of glass or a hydrophilic polymeric latex. The solid may be in various forms such as a rod or a dipstick.

It is preferred that the microbeads are labelled with metal elements and the presence or quantity of the labelled, linked microbeads be determined by X-ray fluorescence analysis. In this preferred embodiment, each microbead represents a SPA reporter which consists of millions of trace atoms which register its presence. The larger amount of the trace elements, or the amplification factor, the easier and more sensitive the reporting becomes.

As each assay is identified by a particular reporting trace element, several assays can be performed in a single procedure by using several corresponding trace elements. Measuring several different trace elements for the reporting function in an X-ray fluorescence analysis requires no more effort than that of an one-item analysis. In a typical x-ray spectrum of microbeads that were irradiated by an x-ray beam, the counting rate of each trace element, or "channel", is proportional to the number of irradiated microbeads with that particular trace element and typical elements are Cr, Fe, Zn and Ba.

Accordingly, when more than one type of dif ferently labelled microbeads are linked to the target mRNA linked onto the solid substrate, the different labelled microbeads can be separately detected simultaneously by X-ray fluorescence characteristic of the different metal element labels. Such a multiple assay enables simultaneous quantitative detection of different gene expressions.

Thus, the invention includes an embodiment wherein the mRNA molecules linked to the solid substrate include different kinds of target mRNA molecules to be assayed, which are assayed by contacting said solid substrate with an aqueous suspension of different varieties of microbeads, each different variety having linked thereto a corresponding different type of gene probe molecules, each type being capable of hybridizing with a particular one of said different kinds of mRNA molecules to be assayed, each variety of microbeads being differently labelled with different metal elements corresponding to each different type of gene probe molecules, thereby selectively linking corresponding, differently labelled microbeads to said different kinds of target mRNA molecules to be assayed which are linked to said solid substrate, and simultaneously and separately determining the presence or quantity of the differently labelled microbeads by analysis of x-ray fluorescence characteristic of each different metal element.

Besides having multiple detecting channels for a simultaneous identification of different target molecules, having a number of X-ray channels provides yet another important advantage, the possibility of internal calibration. In a typical assay, the internal variables such as pH, temperature, incubation time, etc. can be assigned to a particular reference channel to assay a known amount of a certain substance, and its corresponding x-ray reporting channel can be used to calibrate data or counts for all other channels. By using a known channel as an internal standard; the other channels can form an accurate profile of quantitative analyses. This profile can be designed for disease-oriented or problem-oriented analyses with an quantitative internal standard to address a particular biomedical question.

The internal calibration embodiment of the invention comprises adding to the specimen a known quantity of a standard substance which can be assayed by said method and is capable of linking to the solid substrate, including in the aqueous microbead suspension microbeads which are capable of selectively linking to said standard substance and which are correspondingly labelled with metal elements different from the labels of the microbeads which link to the target mRNA molecules to be assayed, simultaneously and separately determining the quantity of the microbeads linked to said target mRNA molecules to be assayed and the quantity of the microbeads linked to said standard substance by analysis of x-ray fluorescence characteristic of the different metal elements, and calibrating the assay of the gene expressions against the assay of the standard substance.

The solid substrate having RNA or DNA polymers which can link with mRNA molecules and the aqueous suspension of labelled microbeads to which gene probes are linked can be provided in accordance with the invention in the form of a kit for assaying gene expressions in aqueous specimens containing mRNA molecules including certain target mRNA molecules to be assayed. The kit of the invention comprises as individual components (a) a solid substrate having conjugated thereto RNA or DNA polymers capable of hybridizing with 3$'$ poly (A) tails of said mRNA molecules; and (b) an aqueous suspension of labelled microbeads to which are linked a plurality of RNA or DNA polymers, each of said linked RNA or DNA polymers being covalently linked at one end to the surface of said labelled micro-bead and being linked at another end to an end of a RNA or DNA gene probe molecule, said gene probe molecule comprising an anti-sense sequence capable of hybridizing with a sense sequence comprised by and characteristic of said certain target mRNA molecules to be assayed.

The microbeads provided with the kit preferably are labelled with metal elements suitable for analysis by x-ray fluorescent techniques. The linking polymers in the kit provided for the solid substrate and the microbeads are as described above.

Furthermore, the kit can be formed from components which enable simultaneous detection of a plurality of different gene expressions. In this embodiment of the kit, above component (b) is an aqueous suspension of different varieties of microbeads, each different variety having linked thereto a corresponding different type of gene probe molecules, each type being capable of hybridizing with a particular one of different kinds of mRNA molecules to be assayed, each variety of microbeads being differently labelled with a different metal element corresponding to each different type of gene probe molecules. In this embodiment, the differently labelled microbeads to which different types of gene probe molecules are linked may be separately packaged as aqueous suspensions of each different type of microbeads.

Also, the kit may include components for providing the assay with an internal standard. In this embodiment the kit includes as individual components: (c) a known quantity of a standard substance which is capable of being assayed in said assaying of gene expressions and is capable of linking to the solid substrate component; and (d) an additional variety of the micro-beads of component (b), included in the aqueous suspension of (b) or packaged as a separate aqueous suspension, wherein the microbeads of said additional variety of microbeads are capable of selectively linking to said standard substance and are correspondingly labelled with metal elements different from the labels of the microbeads which link to the target mRNA molecules to be assayed.

It is preferred to treat the microbeads which have attached gene probes in order to block non-specific binding sites, by treatment or coating of the microbeads with a powdered milk protein (e.g. Carnation non-fat milk powder) or with salmon sperm DNA. A similar product may be used which is free of RNAase (an enzyme which degrades RNA). The substrate can be used with a specimen which is relatively free of RNAase.

The study of oncogenic expressions with the present SPAp system illustrates the present invention, although the method can be equally used for many other genetic expressions.

One of the most important recent advances in the study of oncogenesis has been the realization that various neoplastic transformations are linked, in one way or another, to certain unusual oncogene expressions. There are basically two classes of unusual expressions, one from mutated oncogenes, and another from hyperactive oncogenes. As more viral oncogenes and tumor cell-derived transforming sequences were isolated and molecularly cloned, gene probes for the two dozen or so oncogenes became available for assessing the relationship between their expressions and the malignant diseases. Often, a variety of oncogenic expressions have been found to be elevated as well as mutated in various human tumor cells. Locations called "hot-spots" and elevated expressions have been traced to certain target expressions of the genetic sequences.

Most oncogenes, however, do not routinely participate in the neoplastic transformation. The ras gene family is an example. The human genome contains at least two loci homologous to H-ras-1 and H-ras-2 and K-ras-1 and K-ras-2 genes. Only H-ras-1 and K-ras-2 are functional, whereas the others, H-ras-2 and K-ras-1 correspond to non-functional pseudogenes. H-ras-1, K-ras-2 and N-ras all have similar 4 exon sequnces that code for highly related "protein-21" (p21) with 189 amino acid residues. There are three defined domains of structural significance among the gene products of the known ras genes. A first domain of virtual identity encompasses the first 80 amino acid residues of the p21; the next 80 amino acid residues define a second domain where the different p21 proteins diverge slightly from each other (85% homology between any pair of H-ras-1, N-ras, and C-ras-2). Finally, there is a carboxy-terminal variable region of pronounced divergency possibly for physiologic specificity. The malignant potential of ras proteins can be unleashed by a single amino acid substitution in the highly conserved amino terminal domain for the slight change of p21 protein products. Two "hot-spots" for activation have been detected in human tumor cells and chemically induced malignancy in animals; codon 12 in the first exon and codon 61 in the second exon. In the codon 12 mutation, for example, a flexible hinge region that allows the amino terminus of normal p21 located in the inner side of the plasma membrane to fold into the core of the molecule for GTPase activity disappears in the transforming p21 and leads to a more rigid tertiary structure which can no longer hydrolyze GTP. An assay of sufficient sensitivity would be required to detect this mutation. A greatly increased expression of ras proto-oncogenes, such as those mediated by a retroviral enhancer, can also lead to neoplastic transformation. Analysis for the latter kind of expressions can be assayed in a manner similar to that of the myc proto-oncogenes.

The chromosomal translocation of c-myc locus characteristic of human Burkitt's lymphomas is another important class of neoplastic transformation by proto-oncogenes. The elevated expressions of c-myc rather than qualitative changes appears to be responsible for the oncogenesis in this case. Burkitt's lymphoma cells are characterized by the reciprocal exchange between the end of the long arm of chromosome 8, which harbors the c-myc gene, and chromosomes 14, 2, or 22, which carry the immunoglobulin (Ig) heavy chain, λ light chain, and x light chain locus respectively. Translocation of the c-myc locus leads to its increased expression by the enhancer element of the immunoglobulin loci, although the levels of c-myc transcripts vary considerably from tumor to tumor. Expression of c-myc can be linked to the initial phase $(G_0/G_1)$ of a proliferative response, similar to c-fos and r-fos proto-oncogenes.

In addition to the amplified expression of c-myc, a second genetic event, an appropriate hormonal environment such as pregnancy, is required to initiate the occurrence of tumor development. In terms of human neuroblastomas, N-myc amplification has been implicated in the late stages of the disease and L-myc has been implicated in the small cell lung carcinomas as des-cribed in Seager et. al. N. Engl, J. Med. 313, 1111, 1985. Elevated expression of this family of proto-oncogenes may play a crucial role in the development of a wide spectrum of human malignancies.

While present knowledge of human oncogenesis is far from complete, a family of gene probes can be designed to test the expression of oncogenes with respect to quantitative and qualitative changes. The existing systems of nucleic acid assay require many steps to carry out the task, and diagnosis suffers from variations from tissue to tissue and changes in the assaying environment. Using the SPAp system of the present invention, a profile of assays with high sensitivity, speed, and convenience can be performed with an internal standard for a disease-centered diagnosis, which in turn, can be used to correlate therapy and monitor disease progression.

The method of the invention including the preferred detection by x-ray fluorescence provides the possibility for the simultaneous quantatitive detection of some or even all of as many of 24 oncogenes or more. This can be done with an extremely high sensitivity, so that a small sample will suffice to provide accurate results. This is extremely important where the sample is a tissue specimen such as from cancerous tissue. The use of a small sample enables the sample to be taken from a very small region of the tissue, whereby the assay of gene expressions will be closely related to the region of diseased tissue of interest. In the prior art methods, the assay required taking a very large sample, so that the various gene expressions detected would not necessarily be specific to the tissue region of interest. The alternative in the prior art of obtaining a small sample would require the use of tissue culture to obtain a sufficiently large sample for analysis. This technique, when possible, adds several weeks and other uncertainties to the analysis.

The following example is provided for purposes of illustrating the invention.

## EXAMPLE

### A. SPAp SANDWICH ASSAY

The ras and myc oncogenes are chosen as a model system because of the documented evidence of their implication in human malignances. A sandwich assay is shown in Fig. 1.

Two solid-phase supporters covalently linked to poly (dT) and poly (dC) homopolymers respectively are made. One solid phase (glass) preparation on a dipstick linked to the 5′ end of poly (dT) molecules is used to collect mRNAs from a specimen through the 3′ poly (A) tails of mRNA. Another solid phase (0.2 micrometer microbead) preparation, which contains trace elements as a reporter, Cr for ras and Ti for myc, linked to the 3′ end of poly (dC), is used to form a probe complex through binding of a poly (G)-extended oncogene probe sequence, as indicated in Fig. 1. The chain length of the poly (dT) and the poly (dC) is about 150 to 200 in order to insure accessibility of hybridization with mRNAs and poly (G)-tagged oncogene probes in a solution-phase. Commercially available deoxynucleotide homopolyers are used. Details of the chemical reaction can be found in the above-mentioned text of Maniatis, et al., "Molecular Cloning".

To prepare a poly (G)-extended probe, the chosen oncogene DNA fragment is blunt-end ligated to poly (dG) or poly (dC)homopolymer DNA with about 50 base-pairs such that the 3′ end of poly (dG) is linked to the 5′ end of the anti-sense strand of the probe. The DNA complex is cloned in the Sma I site of the poly-linker region of pGEM vector. SP6 or T7 RNA polymerase transcription of the plasmid precut at the 3′ end of the probe insert will generate the desired poly (dG)-tagged anti-sense probe. This system allows one to prepare a quantitative amount of anti-sense RNA probe for binding to (dC)-tagged solid-phase reporter containing specified elements.

As an alternative approach to prepare a microbead-linked anti-sense probe, ligation of poly (dG) to single-stranded anti-sense DNA can be done. This can be done by synthesizing on oligonucleotide containing 10 to 15 (dC) linked at the 5′ end to 10 to 15 nucleotide of the 3′ end of the anti-sense DNA, which is provided by denaturing the double-stranded probe DNA fragment. The oligonucleotide will serve as a template to facilitate ligation of poly (dG) to the anti-sense strand DNA probe. An advantage of this probe is that it is more resistant to degradation in solution during hybridization.

Also, the probe can be linked to the microbeads in accordance with the embodiment shown in Fig. 2.

### B. SELECTION OF PROBES AND CELL SYSTEMS

Appropriate oncogene probes and cell lines can be chosen for SPA probe hybridization. Various commercially available viral and cellular myc and ras DNA clones are available. Appropriate exon-containing DNA fragments are prepared for the combination described above. Various cell lines and primary cells known to express different levels of myc and ras sequences are used for testing.

## C. RNA PREPARATION AND HYBRIDIATION

Total cellular RNAs are extracted by treatment with guanidine thiocyanate and centrifugation. Coupling of mRNAs to a poly (dT)-coated glass solid phase dip-stick is done by immersing the dipstick in a solution containing a known amount of mRNA extract in the presence of high salt (0.5 N NaCl). Binding of poly (A)-containing RNAs to poly (dT) occurs very rapidly. After 5 to 10 minutes of incubation, the stick is washed by sequential dippings into a moderate salt solution (0.1 N NaCl). The dipstick carrying mRNAs then is ready for hybridization. A poly (dC)-coated microbead reporter labelled with Ti and a poly (G) extended anti-sense probe for myc are mixed similar to the procedure for poly (dT) and poly (A) hybridization. Another poly (dC)- coated microbead reporter, labelled with Cr, and a probe for ras are prepared in the way. The microbead linked probes are collected by centrifugation, washed with moderate salt (0.1 N NaCl) solution and repelleted. Hybridization between dipstick-linked mRNAs and micro-bead reporter-linked anti-sense probe is performed according to conventional conditions (see above Maniatis et al.) with constant agitation. After specified times of hybridization, the dipstick is washed to remove non-specific hybridization and then subjected to x-ray analysis of counts of $K_\alpha$ of Ti and of Cr as described above. Care is taken during washing to prevent destabilization of poly (dT)-poly(A) duplex which has a lower stability than poly (dC)-poly(G) duplex. Controls include known amounts of poly (dC) alone-and plasmid DNA probe-linked microbeads, which are labelled with elements differing from the Ti amd Cr elements used to label the probe-linked microbeads. From the Ti counts the concentration of myc and from the Cr counts the concentration of ras are obtained quantitatively.

The important advantages of the present invention also will be apparent from the following.

Commercial monoclonal antibodies for diagnosis have been available since 1981, and a number of novel, easy-to-use formats employing them have been developed. In comparison gene probe tests are generally more complex. They typically involve nucleic acid extraction, hybridization, and in many non-radioactive test formats, also the equivalent of an enzyme immunoassay. Preparation of a typical gene probe test involves a minimum of tens of hours as follows:

1) sample preparation, purification, and denaturization (minutes to hours);

2) binding to an inert solid support;

3) elimination of non-specific binding sites of the support (many hours);

4) hybridization of bound specimen with a large amount of gene probes in a solid-phase setting (hours to tens of hours);

5) washing to eliminate unhybridized gene probes (many hours);

6) signal development for either autoradiography (hours to days) or enzyme reactions (tens of minutes to hours).

Several gene probe methods recently have been introduced to accelerate or simplify the procedure. One approach uses sequence amplification, such as a "polymerase chain reaction" wherein one-million-fold can be gained in a specific target sequence after 20 to 30 repetitive reaction cycles. There are also approaches of competitive hybridization in a solution-phase where a short labelled signal strand can be displaced from a hybrid when a longer target strand is annealed onto the same hybrid duplex. All these novel systems use either radioactive atoms or enzyme molecules for reporting, each with its unique advantages and difficulties.

Amplication of the myc oncogene can be correlated with, in breast cancer, a shorter time to relapse, a lower survival rate, and a positive number of lymph nodes as disclosed in Slamon et al. SCIENCE 235, 177, 1987. Similar indications include neuroblastoma and small cell lung carcinoma. Major efforts would be required to analyze the oncogenic expressions from a small group of cells, particularly from a fresh specimen. A fast, sensitive and accurate diagnosis such as the SPAp system of the present invention can provide very useful help in oncology, for research as well as for therapy. The following table gives a summary of the present SPAp system in comparison with the Immunogold and Northern Blot procedures:

|  | Immunogold | Northern Blot | SPAp |
|---|---|---|---|
| Detector Sequence | Antibody | DNA Sequence | DNA |
| Reporter Microbeads | Gold Microbead | Radioactive Atoms | Labelled |
| Detector-Reporter Link | Absorption | Chemical | Hybridize |
| Reporting by | Blocking e-beam | Radioactivity | X-Ray Fluorescence |
| Specimen Environment | Vacuum in SEM | Random Solid Bound | Semi-Solution |
| Reporter Sensitivity | Single Bead | Millions of Radioatoms | Thousands of Beads |
| Target-Detector Conjugation | Solid-Liquid | Solid-Liquid | Solution |
| Assay time | Hours | Tens of Hours | Minutes |
| Assay Sensitivity | Affinity Limit | Millions of Senses | Thousands of Senses |

## Claims

1. A method of assaying gene expressions, characterized in that it comprises:
linking mRNA molecules, including certain target mRNA molecules to be assayed, to a solid substrate; contacting said solid substrate with an aqueous suspension of labelled microbeads to which gene probe molecules are linked, said gene probe molecules comprising sequences which hybridize with sequences comprised by and characteristic of said certain target mRNA molecules to be assayed, thereby selectively linking said labelled microbeads to said certain target mRNA molecules to be assayed which are linked to said substrate; separating the solid substrate from microbeads which are not linked to said target mRNA molecules; and determining the presence or quantity of said labelled, linked microbeads; thereby selectively assaying said gene expressions.

2. A method according to claim 1, characterized in that said micro-beads are labelled with metal elements and the presence or quantity of labelled, linked microbeads is determined by x-ray fluorescence analysis.

3. A method according to claim 2, characterized in that the mRNA molecules linked to the solid substrate include different kinds of target mRNA molecules to be assayed, which are assayed by contacting said solid substrate with an aqueous suspension of different varieties of microbeads, each different variety having linked thereto a corresponding different type of gene probe molecules, each type being capable of hybridizing with a particular one of said different kinds of mRNA molecules to be assayed, each variety of microbeads being labelled with different metal elements corresponding to each different type of gene probe molecules, thereby selectively linking corresponding, differently labelled microbeads to said different kinds of target mRNA molecules to be assayed which are linked to said solid substrate, and simultaneously and separately determining the presence or quantity of the differently labelled microbeads by analysis of x-ray fluorescence characteristic of each different metal element.

4. A method according to claim 2 or 3, characterized in that it comprises adding to the specimen a known quantity of a standard substance which can be assayed by said method and is capable of linking to the solid substrate, including in the aqueous microbead suspension microbeads which are capable of selectively linking to said standard substance and which are correspondingly labelled with metal elements different from the labels of the microbeads which link to the target mRNA molecules to be assayed, simultaneously and separately determining the quantity of the microbeads linked to said target mRNA molecules to be assayed and the quantity of the microbeads linked to said standard substance by analysis of x-ray fluorescence characteristic of the different metal elements, and calibrating the assay of the gene expressions against the assay of the standard substance.

5. A kit for assaying gene expressions in aqueous specimens containing mRNA molecules including certain target mRNA molecules to be assayed, characterized in that it comprises as individual components:
(a) a solid substrate having conjugated thereto RNA or DNA polymers capable of hybridizing with 3' poly (A) tails of said mRNA molecules; and
(b) an aqueous suspension of labelled microbeads to which are linked a plurality of RNA or DNA polymers, each of said linked RNA or DNA polymers being covalently linked at one end to the surface of said labelled microbead and being linked at another end to an end of a RNA or DNA gene probe molecule, said gene probe molecule comprising an anti-sense sequence capable of hybridizing with a sense sequence comprised by and characteristic of said certain target mRNA molecules to be assayed.

6. A kit according to claim 5, characteruized in that said microbeads are labelled with metal elements suitable for analysis by x-ray fluorescence.

7. A kit according to claim 5 or 6, characterized in that the polymers linked to the labelled microbeads are poly (X) homopolymers and the gene probe molecules are extended with poly (Y) homopolymers at the end which is linked to said polymers which are linked to said microbeads, wherein:

X is dC and Y is G, X is C and Y is G, X is dG and Y is C, X is G and Y is C, X is dC and Y is dG, X is C and Y is dG, X is dG and Y is dC or X is G and Y is dC.

8. A kit according to claim 6, characterised in that component (b) is an aqueous suspension of different varieties of microbeads, each different variety having linked thereto a corresponding different type of gene probe molecules, each type being capable of hybridizing with a particular one of different kinds of mRNA molecules to be assayed, each variety of microbeads being differently labelled with different metal elements corresponding to each different type of gene probe molecules.

9. A kit according to claim 8, characterized in that the differently labelled varieties of microbeads to which different types of gene probe molecules are linked are separately packaged as aqueous suspensions of each different variety of microbeads.

10. A kit according to claim 6, characterized by further including as individual components:

(c) a known quantity of a standard substance which is capable of being assayed in said assaying of gene expressions and is capable of linking to the solid substrate component; and

(d) an additional variety of the microbeads of component (b), included in the aqueous suspension of (b) or packaged as a separate aqueous suspension, wherein the microbeads of said additional variety of microbeads are capable of selectively linking to said standard substance and are correspondingly labelled with metal elements different from the labels of the microbeads which bind to the target mRNA molecules to be assayed.

FIG.1

FIG.2